# EUROPEAN PATENT APPLICATION

(11) **EP 3 207 860 A1**
(43) Date of publication of application: **23.08.2017**
(21) Application number: 15851550.2
(22) Date of filing: 11.09.2015
(51) Int. Cl.: A61B 1/12, G02B 23/24

(54) **INSERTION-DEVICE CLEANING IMPLEMENT**

(30) Priority: 15.10.2014 JP 2014210878
(71) Applicant: Olympus Corporation, Hachioji-shi Tokyo 192-8507 (JP)
(72) Inventor: YAMAYA, Koji, Hachioji-shi Tokyo 192-8507 (JP)
(74) Representative: Schicker, Silvia
(86) International application number: PCT/JP2015/075909
(87) International publication number: WO 2016/059919

(57) **Abstract**

The distal end portion of an insertion device is fitted into the cylindrical, hollow main body of the washing instrument. A plurality of washing ports are disposed separately from each other on the inner surface of the washing instrument main body. Washing liquids are ejected from the washing ports to a swing base at the distal end portion and its periphery in different directions for washing. When the distal end portion is detached, washing water is sprayed over a shielding wall surface, which is part of the inner surface of the washing instrument main body, to reduce the momentum of the washing liquids and thus prevent the liquids from splashing to the outside.

## Description

### BACKGROUND OF THE INVENTION

### 1.Field of the Invention

The present invention relates to an insertion device washing instrument that is provided with a treatment instrument swing base on the distal end side.

### 2. Description of the Related Art

In general, a treatment instrument having various functions, such as forceps, is inserted through a channel of an inserting section from the main body of an endoscope and extended from an opening formed on the distal end side. Furthermore, there is known a swing mechanism disposed in a treatment instrument receiving chamber in the aperture section provided in a part of an inserting section distal end on its peripheral surface side, to turn, in a desirable direction, a traveling direction of the treatment instrument that extends out from the aperture section. As a typical swing mechanism, a swing base for treatment instrument (or a treatment instrument raising base) is known. The swing base contacts a treatment instrument such as forceps and is raised by operating an operation lever provided on the operation section side to allow the extending direction of the treatment instrument to be changed to a desired direction.

Furthermore, when the inserting section is inserted into a body cavity, a body fluid, resected living tissue pieces, etc. intrude into the receiving chamber from the aperture section in which the swing base is placed, and are attached to the main body of the swing base and its peripheral portion. After end of a treatment, washing and disinfection are essential for an endoscope to prevent infection.

For example, patent literature 1: Jpn. Pat. Appln. KOKAI Publication No. H08-196505, proposes a technology on washing the inside of the distal end portion of an inserting section of an endoscope. In the front surface of the distal end portion of the inserting section of the endoscope, a hole that is an insertion opening which communicates with a treatment instrument receiving chamber and into which a washing instrument is fitted is formed. A connecting portion of the washing instrument fitted into the hole includes a flow channel to feed and suck a washing liquid, and the washing liquid is fed into and sucked from the receiving chamber through the flow channel to remove dirt from the inside of the receiving chamber. In this configuration, since the washing is performed with the connecting portion of the washing instrument directly inserted, a reliable connecting state needs maintaining to prevent a washing liquid in use from leaking and scattering.

Patent literature 1 : Jpn. Pat. Appln. KOKAI Publication No. H08-196505,proposes, as another example, a cover member which covers the whole of the distal end portion and makes it difficult to detach the connecting portion. In the cover member, the connecting portion to be inserted into the hole of an insertion opening is formed to protrude from the inner bottom of the cover member. In other words, if the cover member covers the whole of the distal end portion, the connecting portion is inserted and fixed into the hole. In the cover member, a water drain opening is formed in a position that coincides with the aperture section of the receiving chamber.

In the washing of patent literature 1 described above, a cleaning liquid is fed and sucked through one flow channel in the hole formed on the front surface of the distal end portion of the inserting section and thus the liquid is fed directly to the backside of a swing base from one direction of one place. For this reason, the washing liquid reaches the raising side of a swing base only indirectly, and other portions such as the raising surface of the swing base, the raising shaft and the connection of a traction wire are not washed directly by high water pressure. Thus, there is a case where washing is performed repeatedly depending on the degree of dirty.

Since a drain opening is also formed on the side of the main body of the washing instrument, it is necessary to take measures to prevent the used washing liquid to be drained from scattering. Furthermore, an endoscope to which the washing instrument can be applied needs to be configured to form a hole communicating with a receiving chamber on the front surface of the distal end portion of an inserting section. Thus, the washing instrument cannot be applied to the inserting sections of endoscopes that have been popularized so far.

Accordingly, an object of the present invention is to provide an insertion device washing instrument which makes it difficult to detach the main body of the washing instrument from an insertion device, prevents a washing liquid from scattering to the outside even though the main body is detached, and wash a target portion of the insertion device by spraying a storage storing a swing base with a washing liquid from different directions.

### RIEF SUMMARY OF THE INVENTION

According to an embodiment of the present invention, there is provided an insertion device washing instrument, comprising: a receiving chamber portion which stores a distal end portion of an insertion device in a predetermined fitting position in a tube; a plurality of washing ports disposed separately on an inner surface of the tube to eject a washing liquid to different positions of the distal end portion, which cross a longitudinal axis direction of the receiving portion; and a wall surface provided on an inner surface of the receiving portion to face in a direction in which the washing ports eject the liquid.

Advantages of the invention will be set forth in the description which follows, and in part will be obvious from the description, or may be learned by practice of the invention. The advantages of the invention may be realized and obtained by means of the instrumentalities and combinations particularly pointed out hereinafter.

### BRIEF DESCRIPTION OF THE SEVERAL VIEWS OF THE DRAWINGS

The accompanying drawings, which are incorporated in and constitute a part of the specification, illustrate embodiments of the invention, and together with the general description given above and the detailed description of the embodiments given below, serve to explain the principles of the invention.
FIG. 1 is a schematic diagram of an insertion system according to a first embodiment of the present invention.
FIG. 2A is a top view of a distal end component portion in the first embodiment.
FIG. 2B is a front view of the distal end component portion viewed from arrow 2B shown in FIG. 2A.
FIG. 3 is a cross-sectional view showing a configuration of a swing base inverted in the distal end component portion shown in FIG. 2A.
FIG. 4 is a view showing an external configuration of a washing instrument according to the first embodiment.
FIG. 5 is a view showing a state in which an insertion device is fitted into the washing instrument and cleaned.
FIG. 6 is a view showing a configuration of a cylinder connection member.
FIG. 7 is a view showing a check valve portion in the cylinder connection member.
FIG. 8 is a view of the washing instrument viewed from the front when the insertion device is not fitted into the washing instrument.
FIG. 9A is a cross-sectional view showing the main body of the washing instrument into which the insertion device is fitted.
FIG. 9B is a view of the main body of the washing instrument viewed from the front when the insertion device is fitted into the washing instrument.
FIG. 10 is a view showing an external configuration of the main body of a washing instrument according to a second embodiment.
FIG. 11A is a view showing an external configuration of the main body of a washing instrument according to a third embodiment.
FIG. 11B is a view showing an external configuration of the main body of a washing instrument according to a modification to the third embodiment.
FIG. 12 is a view showing an external configuration of the main body of a washing instrument according to a fourth embodiment.
FIG. 13 is a view showing an external configuration of the main body of a washing instrument according to a fifth embodiment.
FIG. 14 is a view showing an external configuration of the main body of a washing instrument according to a sixth embodiment.
FIG. 15 is a view showing an external configuration of the main body of a washing instrument according to a seventh embodiment.
FIG. 16 is a view showing an external configuration of the main body of a washing instrument according to an eighth embodiment.
FIG. 17A is a cross-sectional view showing a washing instrument according to a ninth embodiment, into which an insertion device is fitted.
FIG. 17B is a view of the washing instrument according to the ninth embodiment viewed from the front.
FIG. 18A is a cross-sectional view of a washing instrument according to a tenth embodiment.
FIG. 18B is a view of the washing instrument according to the tenth embodiment viewed from the front.

### DETAILED DESCRIPTION OF THE PREFERRED EMBODIMENT

Embodiments of the present invention will be described in detail below with reference to the drawings.

### [First Embodiment]

An insertion device washing instrument according to a first embodiment will be described.

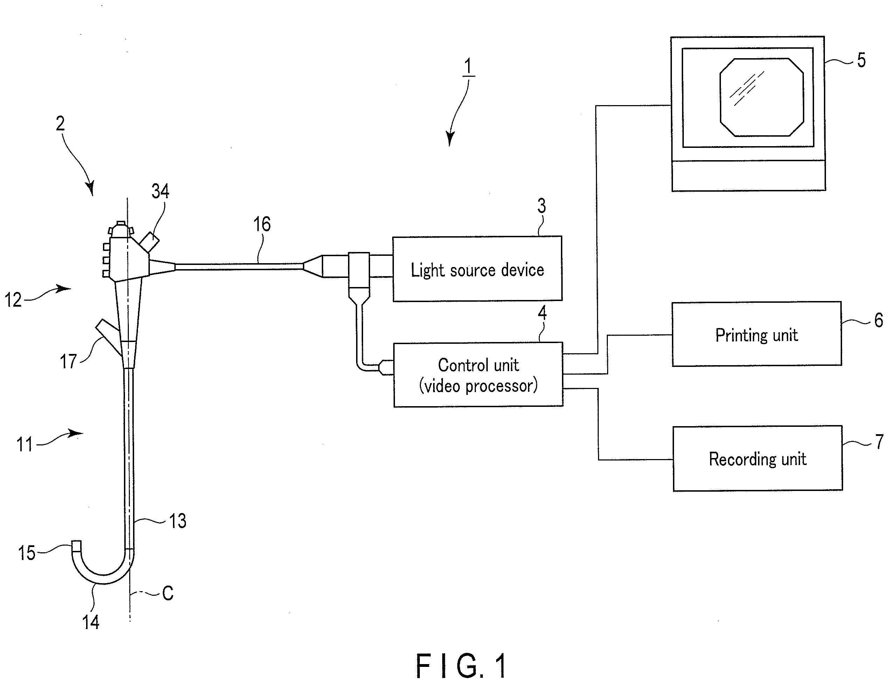

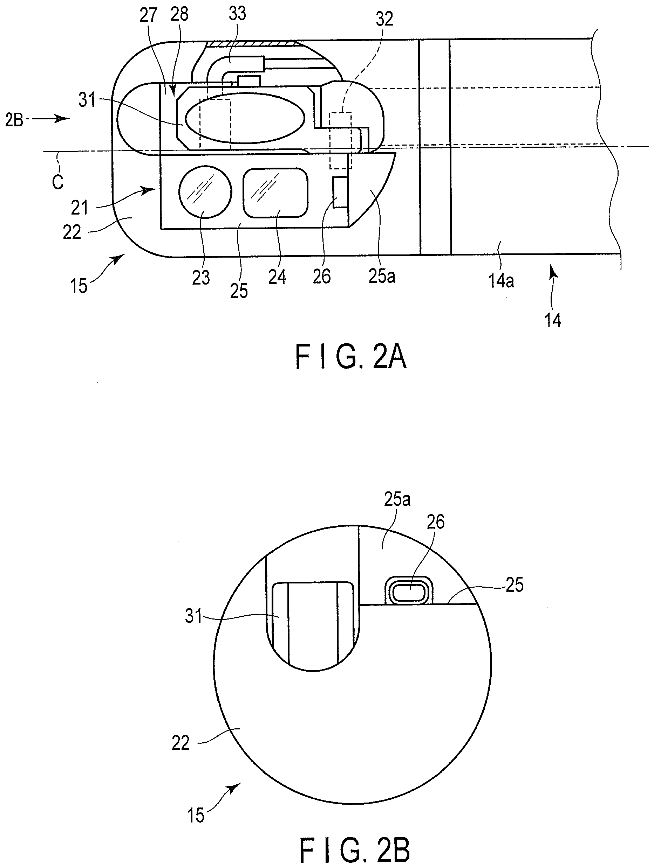

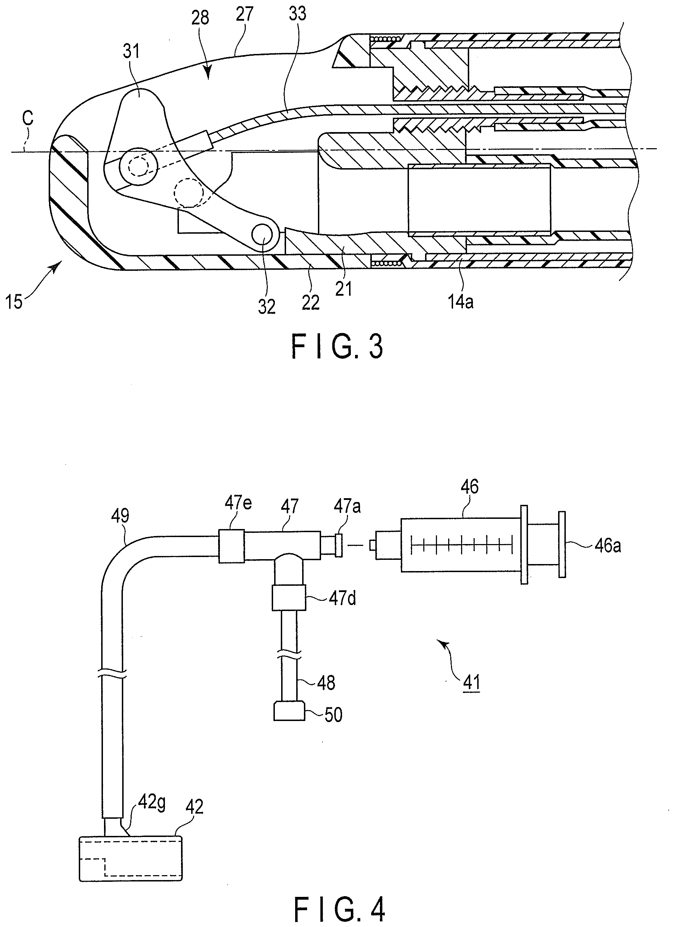

First, as one example of an object to be cleaned by the washing instrument of the first embodiment, a distal end portion including a treatment instrument swing base (referred to as a swing base hereinafter) provided at the distal end of an inserting section of an endoscope apparatus will be described. FIG. 1 is a view showing a system configuration of an endoscope apparatus to be washed. FIG. 2A is a top view of a distal end component portion including the swing base provided at the distal end of an insertion device. FIG. 2B is a view showing an external configuration of the distal end component portion viewed from the front side. FIG. 3 is a cross-sectional view showing a configuration of the swing base inverted in the distal end component portion shown in FIG. 2A.

As shown in FIG. 1, an endoscope apparatus 1 is composed chiefly of an insertion device 2 serving as an endoscope main body, a light source device 3 that supplies illumination light to the insertion device 2, a control section 4 including a video processor which performs image processing for video signals picked up by an imaging unit (not shown) provided at the distal end portion of the insertion device 2, a display section 5 that displays the picked-up observation image and its related information, a printing section 6 that prints image information including the observation image displayed on the display section 5, and a recording section 7 that records the video signals. Here, a medical endoscope apparatus is given as one example of the insertion device, but the insertion device need not be limited to the apparatus. The insertion device 2 can be cleaned by the washing instrument of the first embodiment if it is an industrial endoscope or an insertion device 2, such as a catheter and an overtube, which has neither an illumination optical system nor an observation optical system and is so configured that its opening or portion is put in and taken out of the peripheral surface side.

As shown in FIG. 1, the insertion device 2 is composed chiefly of an inserting section 11 and an operation section 12. The inserting section 11 includes a flexible tube portion 13 connected to the operation section 12, a bending portion 14 connected to the distal end of the flexible tube portion 13, and a distal end constituting section 15 that stores a swing base provided at the distal end of the bending portion 14. The operation section 12 is connected to the light source device 3 and the control section 4 through a universal cord 16. A forceps port 17 is formed between the proximal end portion of the inserting section 11 and the operation section 12 to introduce forceps and the like into the inserting section 11.

As shown in FIG. 3, the distal end constituting section 15, which corresponds to the distal end portion of the insertion device, is connected to the bending portion 14 by fitting a joint ring 14a of the bending portion 14 on the distal end constituting section 15. The distal end constituting section 15 has a laminated structure in which a main body portion 21 is made of metal such as SUS, and its peripheral surface is covered with a cover portion 22 having electrical insulating properties..

As shown in FIGS. 2A and 2B, part of the outer surface of the distal end constituting section 15 is flattened, and the flattened portion is divided into two portions in the longitudinal direction. On one of the two portions, a step 25a is provided on the outer surface to form a flat observation surface 25, and on the other portion, an aperture section 27 is formed. In the observation surface 25, an illumination window portion 23 that is irradiated with illumination light and an observation window portion 24 that includes image pickup elements (not shown), are arranged. In the vicinity of the observation surface 25, a nozzle portion 26 is provided to spray a washing liquid such as saline to clean the illumination window portion 23 and the observation window portion 24 appropriately. This observation surface 25 is a reference position (reference surface) with respect to a rotational direction regulation portion 42h described later.

The aperture section 27 on the other portion is formed in the side surface of the distal end constituting section 15, as shown in FIG. 3. A treatment instrument swing base (referred to as a swing base hereinafter) 31 is rockably placed in a receiving chamber (or a swing base receiving chamber) 28 in the aperture section 27. Character C in FIGS. 1 and FIG. 3 represents a longitudinal direction of the inserting section 11 and the distal end constituting section 15, and indicates the longitudinal axis direction.

The swing base 31 is rotatably supported by a raising shaft 32 and connected to a raising operation portion 34 provided on the operation section 12 by a traction wire 33. When the raising operation portion 34 is operated, the swing base 31 is rotated and raised or inverted. Since, therefore, the swing base 31 and its driving mechanism are placed in the narrow receiving chamber 28, it is hard to remove dirt caused by body fluid and living tissue pieces attached to a shaded portion viewed from the aperture section 27, such as the periphery of the raising shaft 32 and the back surface of the swing base 31. The swing base 31 may be of a type to swing a treatment instrument right and left as well as a type to be raised.

A washing instrument 41 according to the first embodiment will be described below.

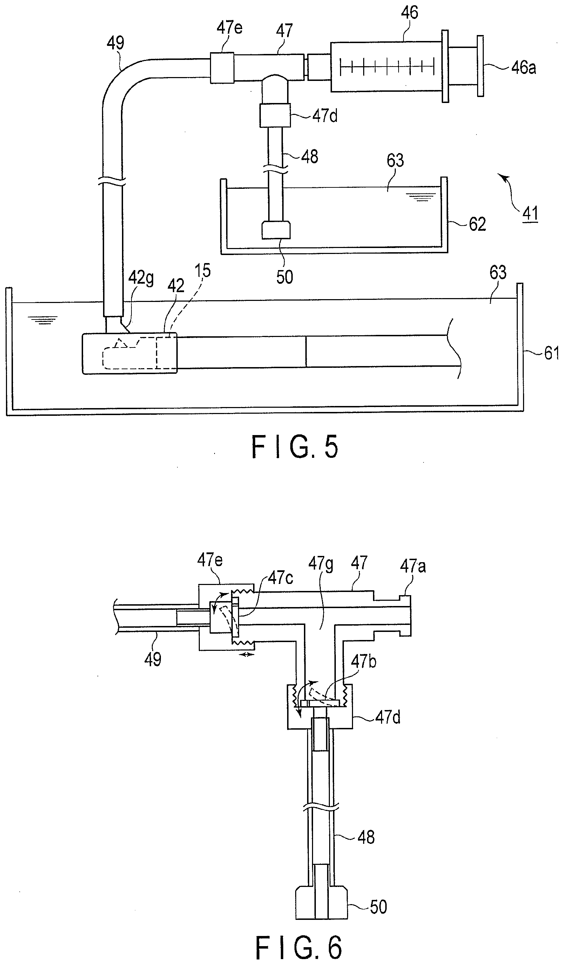

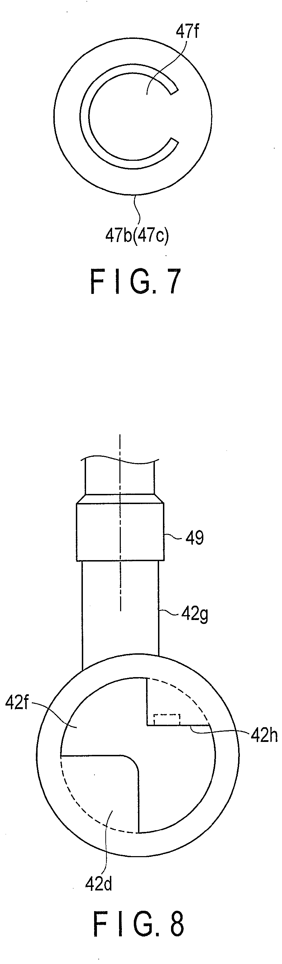

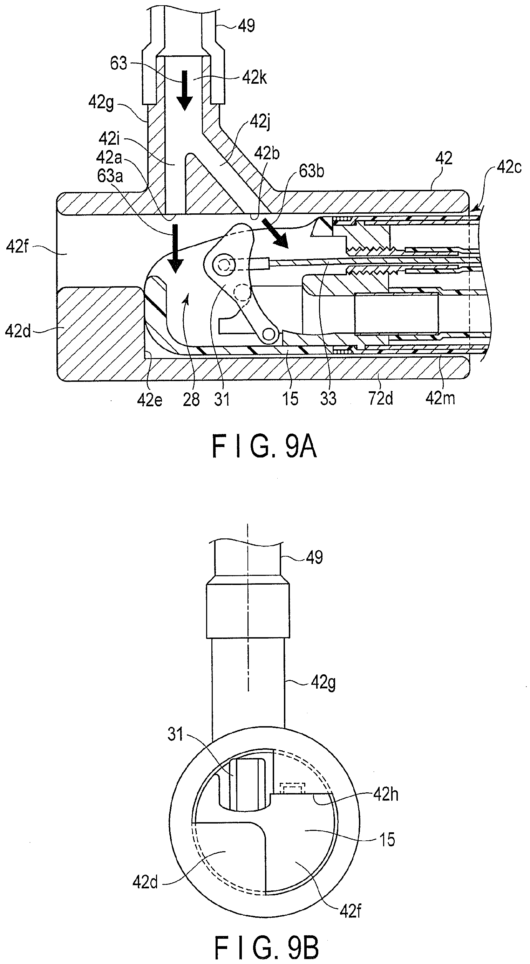

FIG. 4 is a view showing an external configuration of the washing instrument according to the first embodiment, FIG. 5 is a view showing a state in which an insertion device is fitted into the washing instrument and cleaned, FIG. 6 is a view showing a configuration of a cylinder connection member, and FIG. 7 is a view showing a check valve portion in the cylinder connection member. Furthermore, FIG. 8 is a view of the washing instrument viewed from the front when the insertion device is not fitted into the washing instrument, FIG. 9A is a cross-sectional view of the washing instrument into which the insertion device is fitted, and FIG. 9B is a view of the washing instrument viewed from the front when the insertion device is fitted into the washing instrument. Assume that it is the rear of the washing instrument into which the distal end constituting section 15 of the distal end portion of the insertion device is inserted and it is the front thereof into which the distal end portion of the distal end constituting section 15 is brought in the washing instrument described below.

The washing instrument 41 of the first embodiment includes a washing instrument main body 42 having a hollow storage into which the distal end constituting section 15 is fitted, an injection tube 49 connected to a joint portion 42g of the washing instrument main body 42, a syringe connecting portion 47 connected to the injection tube 49, a suction tube 48 extending from a liquid suction end 47d branching from the syringe connecting portion 47, a cylindrical weight 50 which prevents flotation and also serves as a water suction port of the suction tube 48, and a syringe 46 serving as a pump means for causing a washing fluid to flow. If is favorable that the washing instrument 41 described below including the washing instrument main body 42 be made of a transparent member. The use of the transparent member makes it easy to check removal of dirt from outside. If, furthermore, the injection tube 49 and the washing instrument main body 42 are configured detachably to make the washing main body 42 to match the shape of the distal end portion of each of different insertion devices, the washing instrument can be used as a versatile one.

A liquid feed mechanism for feeding a washing liquid by the syringe connecting portion 47 and the syringe 46 will be described with reference to FIG. 4 and FIG. 6.

The syringe connecting portion 47 includes a T-shaped storage chamber (a container that stores a washing liquid temporarily) 47g which branches in three directions. Of the three end portions of the portion 47, two end portions are provided with a liquid suction end 47d and a liquid feed end 47e which are opened, and their insides are provided with check valves 47b and 47c. The other end portion serves as a syringe connecting end 47a to which the syringe 46 is connected. The combination of the syringe 46 and syringe connecting portion 47 functions as a pump.

The check valves 47b and 47c may have a known structure. For example, they can be configured to have a valve portion 47f by forming a C-shaped slit in a circular partition plate made of an elastic member, as shown in FIG. 7. The periphery of one side of the valve portion 47f is applied to the frame of a valve seat to return by elastic force when it is opened and closed. When the valve portion 47f receives pressing force of fluid from one side that is locked by the valve seat, it rotates to cause the fluid to pass. By contrast, when the valve portion 47f receives pressing force of fluid from the other side that is not locked by the valve seat, it is pressed on the frame thereof to stop the fluid from passing.

In the syringe connecting portion 47 shown in FIG. 6, the liquid feed end 47e includes the check valve 47c which is opened by turning the valve portion 47f outward when the inner pressure of the main body of the connecting portion is positive and which is closed by attracting the valve portion to the valve seat when the inner pressure is negative. Furthermore, the liquid suction end 47d includes a check valve 47b which is opened by turning the valve portion 47f inward when the inner pressure of the main body of the connecting portion is negative and which is closed by pressing the valve portion on the valve seat when the inner pressure is positive. This is because the inner pressure of the main body of the connecting portion become negative to cause a washing liquid 63 to flow in by pulling out a plunger 46a of the syringe 46 and it becomes positive to cause the washing fluid 63 to flow out by pushing the plunger 46a. In place of the syringe that feeds a washing liquid, a liquid feed means such as a liquid feed pump can be used.

Washing of the insertion device by the washing instrument 41 of the first embodiment will be described.

FIG. 5 shows a state in which the distal end constituting section 15 of the insertion device 2 is fitted into the washing instrument 41 shown in FIG. 4. The distal end constituting section 15 is fitted into the washing instrument main body 42 of the washing instrument 41 by positioning described later, and the washing instrument main body 42 is set in a drain tray 61. At this time, the washing liquid 63 is put in the drain tray 61 in advance and the washing instrument main body 42 is sunk. Thus, when the washing liquid is vigorously drained from the opening 42f, its momentum can be reduced to prevent the washing liquid 63 from splashing around the drain tray 61. A tray cover can be prepared separately to prevent the washing liquid from splashing. It is favorable that at least the drain tray 61 be made of a transparent member. The use of the transparent member makes it easy to conform the degree of dirt from outside. The external shape of the washing instrument main body 42 is cylindrical. The washing instrument main body 42 not limited to the cylindrical shape but can be shaped like a rectangular box if it has a hollow into which the distal end constituting section 15 can be fitted.

Then, a water suction port 50 of the suction tube 48 is sunk and set in a water suction tray 62 that is filled with the washing liquid 63. The plunger 46a of the syringe 46 is pushed and pulled repeatedly to draw up the washing liquid 63 from the water suction tray 62 through the syringe connection portion 47 and fill the syringe 46. In addition, the washing liquid is fed to the washing instrument main body 42 from the injection tube 49 and ejected in different directions from two washing ports, described later, to clean the receiving chamber 28 including a swing base. The used washing liquid is drained from the receiving chamber 28 to the drain tray 61 through the opening 42f along with deposits. At the same time, the washing liquid flowing out of the receiving chamber 28 is drained into the drain tray 61 while the peripheral portion of the distal end constituting section 15 fitted into the washing instrument main body 42 is being washed.

The washing instrument main body 42 will be described with reference to FIGS. 8, 9A and 9B.

The washing instrument main body 42 of the first embodiment is so configured that one flow channel 42k in the joint portion 42g, which serves as a reception port of the washing liquid 63 and to which the injection tube 49 is connected, branches into two different flow channels 42 i and 42 j in the main body to feed the liquid to first and second washing ports 42a and 42b, respectively. In this example, the first washing port 42a ejects the washing liquid in a direction orthogonal to the longitudinal axis direction of the distal end constituting section 15. The second washing port 42b ejects the washing liquid in an obliquely downward direction from the front of the distal end portion. Naturally, the directions in which the first and second washing ports 42a and 42b eject the washing fluid are not limited but can be modified as appropriate according to their design.

The washing instrument main body 42 is provided with an insertion port 42c to insert the distal end constituting section 15 in the rear, an axial direction regulation portion 42d (a first movement regulation portion), which the distal end constituting section 15 contacts, in the front, a rotational direction regulation portion 42h (a second movement regulation portion), which is opposed to the foregoing observation surface 25 of the distal end constituting section 15, in the upper part of the inner surface, and an opening 42 f to drain the used washing liquid 63 to the outside. The axial direction regulation portion 42d and rotational direction regulation portion 42h constitute a movement regulation portion to set the distal end constituting section 15 in a predetermined fitting position.

In the first embodiment, the washing liquid used for washing can be drained through a clearance 42m between the outer surface of the distal end constituting section 15 and the inner surface of the washing instrument main body 42 as well as the opening 42f. If, furthermore, the amount of washing liquid to be drained is too large to cover the clearance 42m, a concave groove-like gap can be formed in part of the inner surface in the longitudinal axial direction along the inner surface such that the washing liquid can be drained.

As shown in FIG. 9A, the distal end constituting section 15 is inserted into the washing instrument main body 42 from the insertion port 42c, its distal end is brought into contact with a positioning wall 42e of the axial direction regulation portion 42d, and the observation surface 25 and the rotational direction regulation portion 42h are opposed to each other, thereby positioning the distal end constituting section 15 in its axial direction and rotational direction.

The flow channels 42k, 42i and 42j and first and second washing ports 42a and 42b of the washing instrument main body 42 will be described. In the first embodiment, since the washing ports are provided at the distal ends of the linear flow channels, respectively, the flow channels are arranged with an angle from the branch point. The flow channels are not particularly limited if the washing ports are provided to differ in the ejection direction of the washing liquid and the position that the washing liquid reaches. Furthermore, the pressure of the washing liquid to be ejected can be increased by narrowing the diameters of the flow channels 42i and 42j from the flow channel 42k to the first and second washing ports 42a and 42b. The pressure of the washing liquid to be ejected can also be increased by narrowing the first and second washing ports 42a and 42b to cause them to function as a nozzle.

As shown in FIG. 9A, the flow channel 42k branches into two flow channels 42i and 42j in the main body, and there is an inclination due to an arbitrary difference in angle between the flow channels 42i and 42j in the axial direction such that the washing liquid 63 is fed in different directions (at different ejection angles) at the branch point.

The first and second washing ports 42a and 42b are provided separately from each other in such a manner that washing liquids 63a and 63b can be ejected into the receiving chamber 28 from the aperture section 27 of the distal end constituting section 15 at different ejection angles and the inner surface of the main body is opened by the distal end of each of the flow channels. In the first embodiment, during normal washing, the swing base 31 is raised to position its distal end portion between the first and second washing ports 42a and 42b. However, when washing is directed chiefly to the raising surface (surface) of the raising base, the raising base can be tilted completely during the washing.

The first washing port 42a sprays the washing liquid 63 directly within a range from the front side of the receiving chamber 28 of the distal end constituting section 15 (the underside contacting the positioning wall 42e) to the underside of the raised swing base 31 through the bottom surface of the receiving chamber 28. The second washing port 42b sprays the washing liquid 63 directly to the rear and bottom of the receiving chamber 28, the periphery of the raising shaft 32 of the swing base 31, the attaching portion of the traction wire 33, and the like.

Furthermore, the washing instrument main body 42 is opened at both ends. The distal end constituting section 15 of the insertion device such as an endoscope is so configured that not only the opening 42f of the distal end but also the clearance 42m (see FIG. 9A) between the distal end constituting section 15 and the washing instrument main body 42 is enlarged to cause the used washing liquid and dirt to flow to the outside easily. In the first embodiment, two washing ports of the first and second washing ports 42a and 42b are provided, but two or more washing ports can be provided.

As described above, according to the first embodiment, the washing liquids are ejected from the first and second washing ports 42a and 42b disposed separately to the receiving chamber 28 including the swing base 31 in the distal end constituting section 15 of the insertion device 2 in different directions crossing the longitudinal axis of the insertion device 2 (or at different ejection angles), with the result that different portions including the swing base 31 in the receiving chamber 28 are directly sprayed with the washing liquid and cleaned at high pressure. The washing liquids ejected at different angles from a plurality of ports generate a complex water flow in the receiving chamber 28 of the distal end constituting section 15 to achieve a more effective washing. Incidentally, the washing liquids can be ejected from the washing ports at different locations at the same angle (in parallel).

Furthermore, even though the washing instrument main body 42 is displaced unintentionally, the distal end constituting section 15 is covered cylindrically and thus the washing liquids ejected from the first and second washing ports 42a and 42b impinge on part of the inner surface (wall surface) of the washing instrument main body 42 and flow out in the longitudinal direction, with the result that the washing liquids do not directly splash the operator. Here, the inner surface on which the washing liquids impinge is equivalent to the fluid shielding wall of a third embodiment, which will be described later. Furthermore, even though the used washing liquid is discharged in water by performing a washing washing tool body 42 submerged in the water is discharged into the water, when the insertion device is cleaned by the washing instrument main body 42 sunk in water and the washing liquid used for the washing flows into the water, even though the distal end constituting section 15 is removed from the washing instrument main body 42, the used washing liquid can be prevented from scattering to the outside. If, furthermore, the injection tube 49 and the washing instrument main body 42 are configured detachably and only washing instrument main bodies are made to conform to the shapes of distal end portions of various insertion devices, a versatile washing instrument can be achieved. Moreover, only the used washing instrument main body 42 can be scrapped.

### [Second Embodiment]

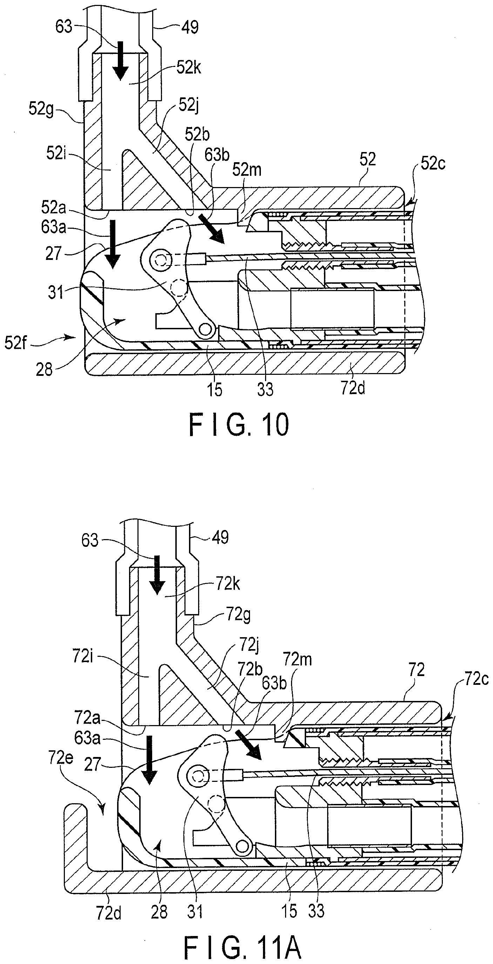

FIG. 10 is a view showing an external configuration of the washing instrument main body of an insertion device according to a second embodiment. In the second embodiment, the axial direction regulation portion is changed in the first embodiment described above. The structural portions other than the axial direction regulation portion are equivalent to those of the first embodiment described above.

The axial direction regulation portion of the foregoing first embodiment is provided on the front side of the washing instrument main body and its distal end is brought into contact with a positioning wall 42e of the axial direction restriction portion 42d to position the rotation direction and axial direction of the distal end constituting section 15. As the insertion device 2 , however, there is an insertion device having a configuration that is unsuitable to bring the distal end of the distal end constituting section 15 into contact, such as when the distal end is provided with some functions (e.g., a front image pickup unit to pick up an image of the front during insertion and movement). In the second embodiment, therefore, the set position of the axial direction regulation portion is changed and the shape thereof is changed according to the set position.

The cleaning instrument main body 52 of the second embodiment is shaped like a cylinder which is opened on its front and rear side, and provided with a joint portion 52g to which an injection tube 49 is connected. One flow channel 52k in the joint portion 52g branches into flow channels 52i and 52j at two different angles in the main body to feed a washing liquid to each of the first and second washing ports 52a and 52b. The first and second washing ports 52a and 52b are provided separately from each other in such a manner that the washing liquids 63a and 63b can be ejected into the receiving chamber 28 from the aperture section 27 of the distal end constituting section 15 at different ejection angles and the inner surface of the main body is opened by the distal end of each of the flow channels.

On the inner surface of the washing instrument main body 52, when the distal end constituting section 15 is inserted from an insertion port 52c, a projection-like axial direction regulation portion 52m is provided such that the edge of the aperture section 27 on the proximal end side abuts the distal end component portion. The position of the axial direction regulation portion 52m is set such that the washing liquids 63a and 63b are ejected from the first and second washing ports 42a and 42b of the foregoing first embodiment to the equivalent position in the receiving chamber 28 at the same ejection angle. Since, furthermore, the front of the washing instrument main body 52 is not provided with an axial direction regulation portion, its entire surface is opened and used as an opening portion 52f through which the used washing liquid 63 is drained to the outside.

In addition to the advantage of the foregoing first embodiment described above, the washing instrument of the second embodiment can be applied to even an insertion device that is unsuitable to bring the distal end portion of the distal end component portion 14 of the insertion device 2 into contact because the axial direction regulation portion is moved from the front of the washing instrument main body to the inside thereof and the distal end portion of the distal end constituting section 15 need not be brought into contact with the opening at the time of positioning. In addition, the axial direction regulation portion is excluded from the front of the washing instrument main body and thus the washing instrument can be shortened in its longitudinal direction. Similarly, when the used washing liquid is drained, the entire surface of the washing instrument main body 52 serves as an opening to drain the washing liquid. Thus, the washing instrument of the second embodiment can be applied even though the amount of washing liquid to be drained increases.

### [Third Embodiment]

FIG. 11A is a view showing an external configuration of the washing instrument main body of an insertion device according to a third embodiment. The third embodiment has a configuration in which part of the fluid shielding wall extends to the front in addition to the configuration of the washing instrument main body 52 of the second embodiment described above. In the third embodiment, the configurations excluding the shape of the fluid shielding wall are equivalent to those of the foregoing second embodiment.

In the third embodiment, part of a fluid shielding wall 72d extends semicircularly to the lower front of the washing instrument main body 72 with a space 72e. Usually, the fluid shielding wall 72d decreases the used washing liquid drained from the aperture section 27 of the distal end constituting section 15 by applying the liquid onto the fluid shielding wall to cause the drain tray (not shown) to contain the liquid. Even though the distal end constituting section 15 is displaced or detached from the fitting position of the washing instrument main body 72 unintentionally, the washing liquids 63a and 63b ejected from the first and second washing ports 71a and 71b impinge on part of the inner surface of the washing instrument main body 71 and thus the momentum of the washing liquids is reduced. Then, the washing liquids impinge are reflected and scattered on the inner surface and flow out from the opening of the front. The momentum of the washing liquids is further reduced by the semicircular fluid shielding wall 72d, and they are not splashed, with the result that the washing liquids do not splash the operator.

As described above, according to the third embodiment, the fluid shielding wall 72d extends to the lower front of the washing instrument main body 72 to make it possible to reduce the momentum of the used washing liquid to be drained and thus prevent the used washing liquid from splashing to the outside.

Furthermore, even though the distal end constituting section 15 is displaced or detached from the fitting position of the washing instrument main body 72 unintentionally, the washing liquids 63a and 63b ejected from the first and second washing ports 72a and 72b impinge on the fluid shielding wall 72d that is the bottom portion of the washing instrument main body 72 to reduce the momentum of the washing liquids and drain the washing liquids from the opening 72f to the outside, with the result that the washing liquids do not splash the operator.

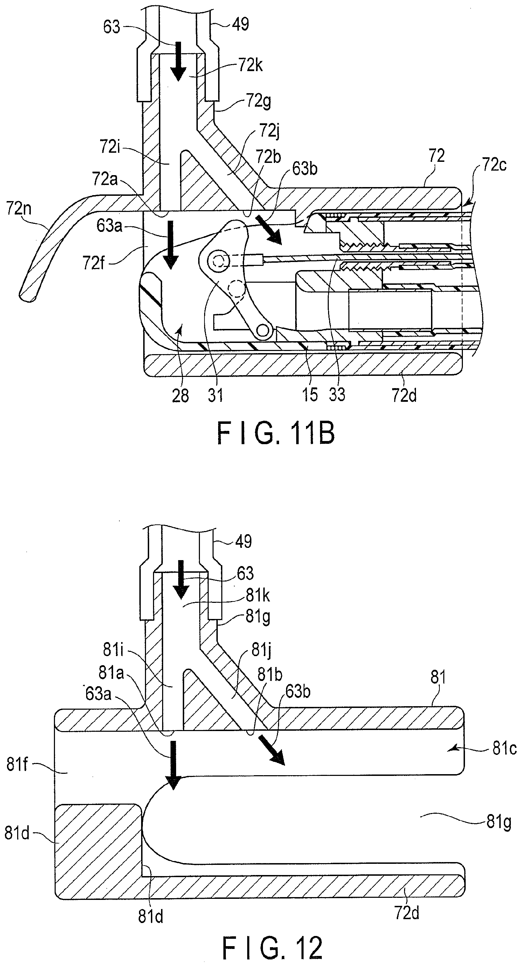

FIG. 11B is a view showing an external configuration of the main body of a washing instrument according to a modification to the third embodiment. According to this modification, in the washing instrument main body 52 of the third embodiment described above, not the fluid shielding wall 72d part of which is formed to rise from the lower front side of the washing instrument main body 52, but an eaves-shaped splash prevention wall 72n which falls obliquely downward from the upper front side is provided against the fluid shielding wall 72d of the second embodiment. In the third embodiment, the configuration other than the splash prevention wall 72n is equivalent to that of the second embodiment described above.

According to this modification, in addition to the advantage of the second embodiment, the splash prevention wall 72n is able to reduce the momentum of the used washing liquid to be drained from the aperture section 27 of the distal end constituting section 15 so as to cover the washing liquid from above and then discharge the washing liquid into the drain tray (not shown). Furthermore, even though the distal end constituting section 15 is detached from the washing instrument main body 72, the momentum of washing liquids splashed by the fluid shielding wall 72d is reduced by the splash prevention wall 72n to prevent the washing liquids from splashing upward onto the operator. Since, moreover, the splash prevention wall 72n is protruded obliquely downward like eaves, the flow of the washing liquid to be drained can be prevented from stopping while reducing the momentum of the washing liquid.

### [Fourth Embodiment]

FIG. 12 is a view showing an external configuration of the washing instrument main body of an insertion device according to a fourth embodiment.

In addition to the configuration of the washing instrument main body 42 of the first embodiment described above, the fourth embodiment has the configuration in which a cutaway portion 81g is provided on the side of the washing instrument main body. In the fourth embodiment, the configuration other than the cutaway portion is equivalent to that of the first embodiment described above.

In the fourth embodiment, a U-shaped cutaway portion 81g, which extends from an insertion port 81c at the rear end to a positioning wall 81e of an axial direction regulation portion 81d, is formed on at least one side of a washing instrument main body 81. The fourth embodiment includes a region opposed to washing liquids 63a and 63b ejected from first and second washing ports 81a and 81b, and part of the washing instrument main body 81 is left like a rectangle. Assume here that a surface on which the washing liquids 63a and 63b to be ejected impinge is the fluid shielding wall 72d.

With the above-described configuration, even though the distal end constituting section 15 is displaced from the fitting position of the washing instrument main body 81, the washing liquids 63a and 63b ejected from the first and second washing ports 81a and 81b impinge on part of the washing instrument main body 81 to reduce their momentum and then flow toward the side surface from the cutaway portion 81g; thus, the washing liquids do not splash the operator. Since, furthermore, the washing instrument main body 81 is sunk in the water for washing, the used washing liquids are drained in the water. Even though the distal end constituting section 15 is detached from the washing instrument main body 81, the used washing liquids can be prevented from splashing to the outside.

### [Fifth Embodiment]

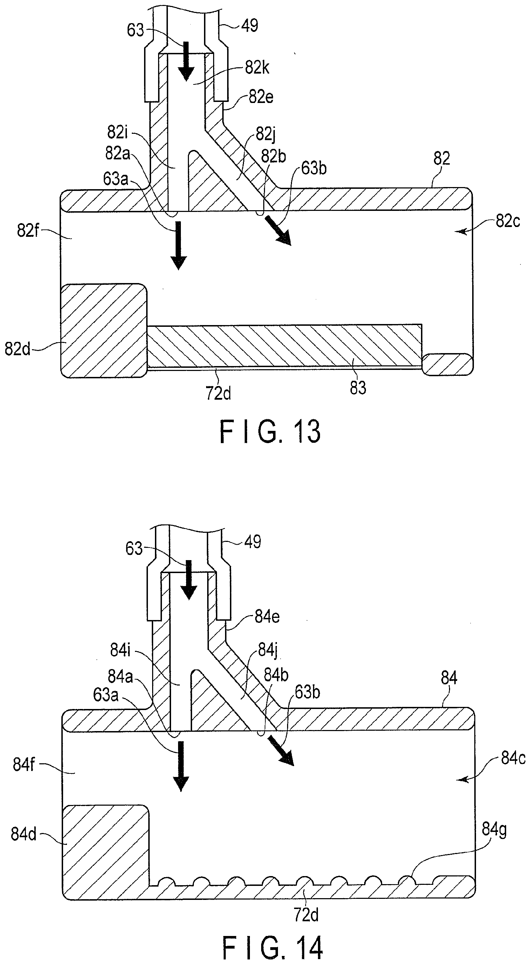

FIG. 13 is a view showing an external configuration of the washing instrument main body of an insertion device according to a fifth embodiment.

In addition to the configuration of the washing instrument main body 42 of the first embodiment described above, a fluid attenuation portion 83 that is made of a mesh member is provided in a region on which the washing liquids 63a and 63b ejected from first and second washing ports 82a and 82b of a washing instrument main body 82 impinge. In the fifth embodiment, the configuration other than the fluid attenuation portion is equivalent to that of the first embodiment described above. Here, the fluid attenuation portion 83 also serves as a fluid shielding wall 72d.

With the above-described configuration, even though the distal end constituting section 15 is displaced from the fitting position of the washing instrument main body 81, some of the washing liquids 63a and 63b ejected from the first and second washing ports 82a and 82b pass through the fluid attenuation portion 83 (fluid shielding wall 72d) to reduce their momentum and then are drained to the outside from the opening 72f; thus, the washing liquids do not splash the operator. Since, furthermore, the washing instrument main body 82 is sunk in the water for washing, the used washing liquids are drained in the water. Even though the distal end constituting section 15 is detached from the washing instrument main body 82, the used washing liquids can be prevented from splashing to the outside.

### [Sixth Embodiment]

FIG. 14 is a view showing an external configuration of the washing instrument main body of an insertion device according to a sixth embodiment.

In addition to the configuration of the washing instrument main body 42 of the first embodiment described above, a plurality of uneven portions 84g are formed in a region on which the washing liquids 63a and 63b ejected from first and second washing ports 84a and 84b of a washing instrument main body 84 impinge. Here, the uneven portions 84g also serve as a fluid shielding wall 72d. In the sixth embodiment, the configuration other than the uneven portions is equivalent to that of the first embodiment described above.

With the above-described configuration, even though the distal end constituting section 15 is displaced from the fitting position of the washing instrument main body 84, the washing liquids 63a and 63b ejected from the first and second washing ports 84a and 84b impinge on the uneven portion 84g (fluid shielding wall 72d) and diffuse in various directions to reduce their momentum and then are drained to the outside from an opening 82f; thus, the washing liquids do not splash the operator. The uneven portion 84g has the function of preventing the distal end constituting section 15 from being displaced or detached if it is brought into point contact with the distal end constituting section 15.Since, furthermore, the washing instrument main body 84 is sunk in the water for washing, the used washing liquids are drained in the water. Even though the distal end constituting section 15 is detached from the washing instrument main body 84, the used washing liquids can be prevented from splashing to the outside.

### [Seventh Embodiment]

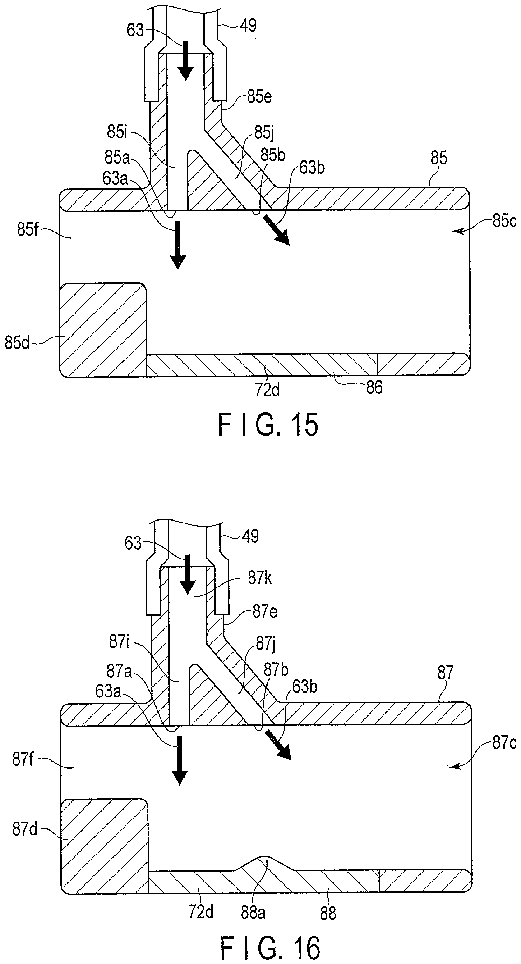

FIG. 15 is a view showing an external configuration of the washing instrument main body of an insertion device according to a seventh embodiment.

In addition to the configuration of the washing instrument main body 42 of the first embodiment described above, a fluid lessening portion 86 that is made of an elastic member is provided in a region on which the washing liquids 63a and 63b ejected from first and second washing ports 85a and 85b of a washing instrument main body 85 impinge. In the seventh embodiment, the configuration other than the fluid lessening portion is equivalent to that of the first embodiment described above. Here, the fluid lessening portion 86 also serves as a fluid shielding wall 72d.

With the above-described configuration, even though the distal end constituting section 15 is displaced from the fitting position of the washing instrument main body 85, the washing liquids 63a and 63b ejected from the first and second washing ports 85a and 85b impinge on the fluid lessening portion 86 (fluid shielding wall 72d) to lessen and reduce their momentum by elastic force and then are drained to the outside from an opening 85f. Thus, the drained washing liquids do not splash the operator. Since, furthermore, the washing instrument main body 85 is sunk in the water for washing, the used washing liquids are drained in the water. Even though the distal end constituting section 15 is detached from the washing instrument main body 85, the used washing liquids can be prevented from splashing to the outside.

### [Eighth Embodiment]

FIG. 16 is a view showing an external configuration of the washing instrument main body of an insertion device according to an eighth embodiment.

In the eighth embodiment, a fluid lessening portion 88 to which the blocking function of a convex portion 88a is added is provided in the fluid lessening portion 86 of the washing instrument main body 85 of the seventh embodiment described above. In the eighth embodiment, the configuration other than the convex portion is equivalent to that of the seventh embodiment described above. Here, the fluid lessening portion 88 also serves as a fluid shielding wall 72d.

The convex portion 88a is at least one convex portion protruded like a mountain or a convex portion protruded like a ring or like a C-shaped fold.

With the above-described configuration, the fluid lessening portion 88 (fluid shielding wall 72d) functions as a blocking member made of an elastic member for the distal end constituting section 15 and is hard to detach from the washing instrument main body 87, in addition to the advantage of the seventh embodiment described above.

### [Ninth Embodiment]

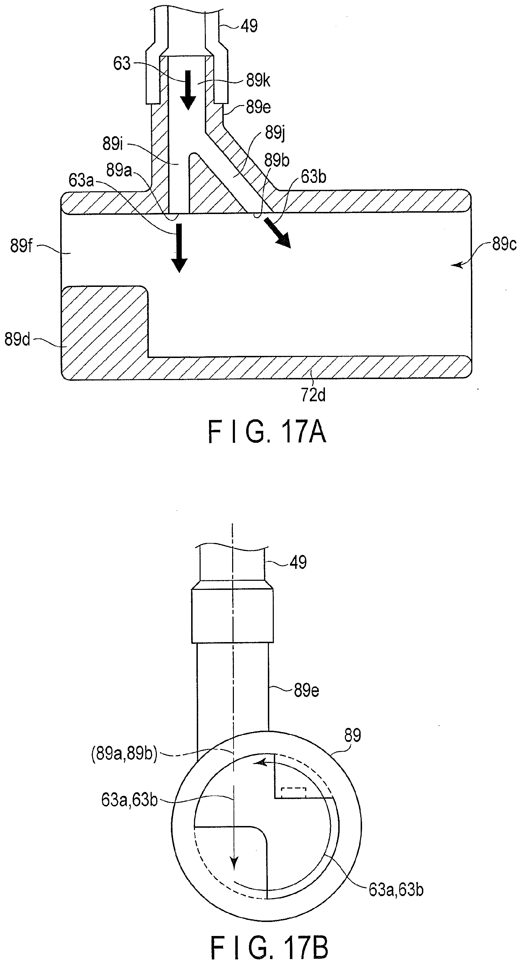

FIG. 17A is a cross-sectional view showing a washing instrument according to a ninth embodiment, and FIG. 17B is a view of the washing instrument according to the ninth embodiment, viewed from the front. According to the ninth embodiment, in the configuration of the washing instrument main body 42 of the first embodiment described above, the positions of the first and second washing ports are moved outside the diameter from the longitudinal axis of the washing instrument.

As shown in FIG. 17A, the first and second washing ports 85a and 85b, or the joint portion 89g is moved outside the diameter from the longitudinal axis of the washing instrument within a range in which the ejected washing liquids 63a and 63b are put into the aperture section 27 of the distal end constituting section 15.

As shown in FIG. 17B, even though the distal end constituting section 15 is detached from the fitting position of the washing instrument main body 89 by moving the first and second washing ports 89a and 89b, the washing liquids 63a and 63b ejected from the first and second washing ports 89a and 89b flow along the inner surface of the washing instrument main body 89 and then pass the first and second washing ports 89a and 89b, as indicated by the arrows. When the washing liquids pass the first and second washing ports, a washing liquid 63 that has flowed along the inner surface and a washing liquid 63 that has been ejected newly impinge on each other and their momentum can be reduced.

Since, therefore, the momentum of the washing liquids is reduced and lessened and the washing liquids are drained to the outside from the opening 89f, the washing liquids do not splash the operator. Furthermore, the washing instrument main body 89 is sunk in the water for washing, and the used washing liquids are drained in the water. Even though the distal end constituting section 15 is detached from the washing instrument main body 89, the used washing liquids can be prevented from splashing to the outside.

### [Tenth Embodiment]

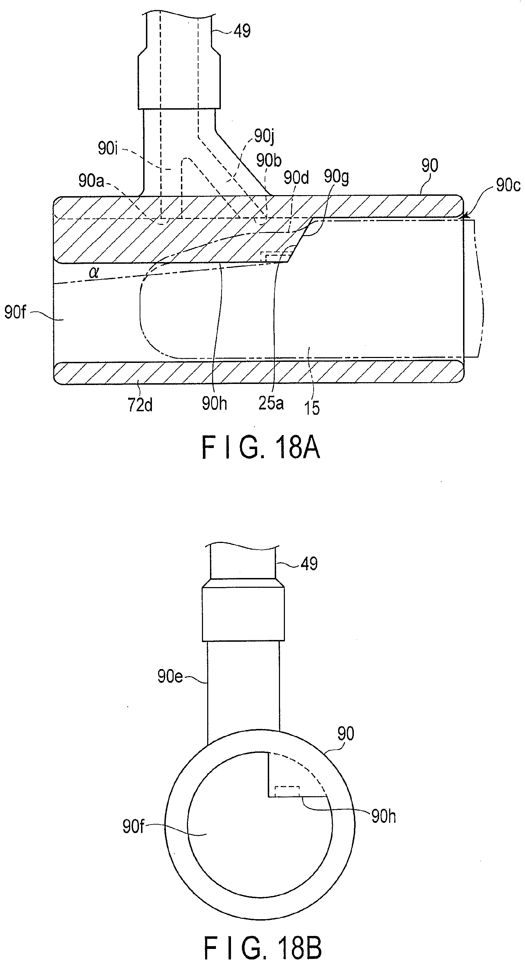

FIG. 18A is a cross-sectional view of a washing instrument into which an insertion device according to a tenth embodiment is fitted, and FIG. 18B is a view of the washing instrument according to the tenth embodiment, viewed from the front. In the tenth embodiment, a positioning portion 90d having the functions of the axial direction regulation portion 42d and rotational direction regulation portion 42h in the configuration of the washing instrument main body 42 of the first embodiment described above, is provided. In the tenth embodiment, the configuration other than the positioning portion is equivalent to that of the first embodiment described above.

In the tenth embodiment, the foregoing positioning portion 90d is provided by forming the axial direction regulation portion 42d and rotational direction regulation portion 42h integrally with each other. As shown in FIG. 18A, the positioning portion 90d has an inclined surface 90g, which contacts a step 25a that differs in level from the outer surface, on the proximal end side of the observation surface 25 of the distal end constituting section 15 and extends from below the inclined surface 90g to the front surface and faces the observation surface 25. This inclined surface 90g functions as an axial direction regulation portion. Accordingly, as shown in FIG. 18B, the opening 90f is formed on the front surface of the washing instrument main body 90 and does not have any equivalent for the axial direction regulation portion 42d shown in FIG. 9A

Furthermore, the lower surface (rotation regulation surface) 90h of the positioning portion 90d functions as a rotational direction regulation portion. As shown in FIG. 18B, the rotation regulation surface 90h is set such that its distal end side approaches the observation surface 25 at angle difference α. If, furthermore, the rotation regulation surface 90h is inclined in the distal end direction at angle difference α to narrow the space in which the distal end constituting section 15 is stored, the washing instrument main body 90 can regulate the distal end constituting section 15 of the insertion device more reliably by the rotational direction and the axial direction.

With the above-described configuration, even though the distal end constituting section 15 is displaced or detached from the fitting position of the washing instrument main body 90, the washing liquids ejected from first and second washing ports 90a and 90b impinge on the inner surface of the washing instrument main body 90 and circulates, and also impinge on the rotation regulation surface to reduce the momentum of the washing liquid 63 and then are drained to the outside from the opening 90f. Thus, the washing liquids do not splash the operator. Since, furthermore, the washing instrument main body 95 is sunk in the water for washing, the used washing liquids are drained in the water. Even though the distal end constituting section 15 is detached from the washing instrument main body 90, the used washing liquids can be prevented from splashing to the outside.

According to the present invention, there can be provided a washing instrument for an insertion device which makes it difficult to detach the main body of the washing instrument from the insertion device, prevents a washing liquid from scattering to the outside even though the main body is detached, and cleans a target portion of the insertion device by spraying a receiving chamber storing a swing base with a washing liquid from different directions.

Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as defined by the appended claims and their equivalents.

## Claims

1. An insertion device washing instrument, comprising:
a receiving portion which stores a distal end portion of an insertion device in a predetermined fitting position in a tube;
a plurality of washing ports disposed separately on an inner surface of the tube to eject a washing liquid to different positions of the distal end portion, which cross a longitudinal axis direction of the receiving portion; and
a wall surface provided on an inner surface of the receiving portion to face in a direction in which the washing ports eject the liquid.

2. The insertion device washing instrument according to claim 1, wherein the washing ports feed the washing liquid in a direction orthogonal to an insertion direction of the receiving portion.

3. The insertion device washing instrument according to claim 1, wherein the receiving portion includes a movement regulation portion which locates the distal end portion in a position in which the liquid ejected from the plurality of washing ports directly impinge on a washing target portion of the distal end portion to be stored.

4. The insertion device washing instrument according to claim 3, wherein:
the distal end portion includes an opening which is formed in the outer surface of the distal end portion stored in the receiving portion, and a swing base which rocks a treatment instrument extending outward; and
the movement regulation portion locates the distal end portion in a position in which the liquid ejected from the washing ports directly impinge on the opening and the swing base.

5. The insertion device washing instrument according to claim 3, wherein the movement regulation portion includes a first movement regulation portion which prevents the distal end portion from moving in the longitudinal axis direction of the receiving portion and a second movement regulation portion which prevents the distal end portion from rotating in a rotational direction on a longitudinal axis of the receiving portion from the fitting position.

6. The insertion device washing instrument according to claim 1, wherein the plurality of washing ports eject the liquid to different positions in the receiving portion.

7. The insertion device washing instrument according to claim 4, wherein one of different directions in which the plurality of washing ports eject the liquid in the opening is a direction orthogonal to an insertion direction of the receiving portion.
